# EUROPEAN PATENT APPLICATION

(11) **EP 4 382 173 A1**
(43) Date of publication of application: **12.06.2024**
(21) Application number: 22211999.2
(22) Date of filing: 07.12.2022
(51) Int. Cl.: A61Q 5/06, A61Q 5/04, A61K 8/06, A61K 8/365, A61K 8/49, A61K 8/34, A61K 8/73, A61K 8/891, A61K 8/04, A61K 8/44

(54) **AQUEOUS COMPOSITION FOR SEMI-PERMANENT STRAIGHTENING KERATIN FIBERS ESPECIALLY HUMAN HAIR**

(71) Applicant: Kao Germany GmbH, 64297 Darmstadt Hessen (DE)
(72) Inventor: ILHAM, stephanie, 64297 Darmstadt (DE); BAUER, Peter, 64297 Darmstadt (DE); BREAKSPEAR, Steven, 64297 DARMSTADT (DE); NÖCKER, Bernd, 64297 DARMSTADT (DE)

(57) **Abstract**

The present invention relates to an aqueous composition for semi-permanent straightening keratin fibers, especially human hair comprising one or more carboxylic acids and one or more amino acids and having a pH 4.0 or less.

## Description

The present invention relates to an aqueous composition for the semi-permanent straightening of keratin fibers, especially human hair, comprising one or more carboxylic acids and one or more amino acid and having a pH 4.0 or less.

Permanent hair straightening, via a reductive and then oxidative process, has long been known. To keratin fibers, such as human hair, are applied aqueous composition comprising a reducing agent for cleaving the disulfide bonds and the cleaved bonds are oxidatively rearranged by using a composition comprising oxidizing agent. Such kinds of processes are relatively damaging to keratin fibers and, therefore, users have been looking for less damaging alternatives, giving the possibility of repeated usage.

Recently, there have been new products made available by the applicant not involving reductive and oxidative process. These products are based on carboxylic acids and have relatively low pH values; effective straightening is realized by heating up the hair to approximately 200°C with a straightening iron. Hair straightened by such a process maintains a straight shape for up to 6 months. Such kinds of processes have been subject to various patent applications from the current applicant, such as EP2961375. Even whenthe process does not involve the use of strongly alkaline reducing compositions, and the straightening effect has been found very satisfactory, the fibers still lose certain physico-chemical properties and, therefore, there is still a need for more fiber friendly compositions and processes. The fiber properties such, as elasticity are been known to be very important for natural appearance of hair.

The inventors of the present invention have unexpectedly found out that addition of one or more amino acids into semipermanent straightening compositions, based on carboxylic acids at lower pH values, prevents loss of hair elasticity and hair straightened with such compositions feels smoother, more elastic, and has a natural like suppleness.

WO 2015/086230A1 discloses hair straightening compositions and processes, therefore, using carboxylic acid, namely glyoxylic acid and its salts. FR 3095756 discloses hair straightening compositions under strong alkaline conditions which may comprise amino acids. WO 2017/041904 discloses straightening and dyeing compositions and processes wherein the straightening is carried out with a composition comprising glyoxylic acid. In none of the documents is a straightening based on one or more carboxylic acid and one or more amino acid is not disclosed.

Accordingly, the first object of the present invention is an aqueous composition for the semi-permanent straightening of keratin fibers, especially human hair, comprising at least one carboxylic acid of the formula (I) and/or a hydrate thereof and/or a salt thereof:

R-CO-COOH Formula (I)

wherein R is selected from hydrogen, COOH, CN, optionally substituted C₁-C₁₀ alkyl, optionally substituted C₂-C₁₀ alkenyl, optionally substituted C₂-C₁₀ alkynyl, optionally substituted C₃-C₁₀ cycloalkyl, optionally substituted C₆-C₁₀ aryl or a 5-10-membered, optionally substituted heteroaryl group, wherein the optional substituents of the alkyl group are selected from halogen, hydroxyl, amino and C₁-C₄ alkoxy, and the optional substituents of the other groups are selected from halogen, hydroxyl, amino, C₁-C₄ alkyl and C₁-C₄ alkoxy; and
one or more amino acids, and
the composition has a pH of 4.0 or less.

The composition may be a one-part composition comprising both ingredients or a two-part composition comprising Parts A and B which are kept separate and mixed to form a homogeneous composition immediately before application onto hair, wherein the Part A comprises at least one carboxylic acid of the formula (I) and/or a hydrate thereof and/or a salt thereof, and the Part B comprises one or more amino acids.

The second object of the present invention is the use of the composition for straightening keratin fibers, especially human hair.

The third object of the present invention is on the method of straightening keratin fibers especially human hair characterized in that it comprises the steps of
- application of the composition according to any of the claims 1 to 11,
   - leaving on the hair for a period of 1 to 120 min,
   - optionally rinsing off the hair,
   - drying the hair,
   - treating the hair with an iron having a surface temperature of 180 ± 50°C; and
   - optionally rinsing off the hair and/or shampooing the hair and drying.

Further object of the present invention is a kit comprising the composition of the present invention and a straightening iron.

The semi-permanent straightening composition comprises at least one carboxylic acid of the following formula (I) as the active component for achieving the straightening effect:

R-CO-COOH Formula (I)

wherein R is selected from hydrogen, COOH, CN, optionally substituted C₁-C₁₀ alkyl, optionally substituted C₂-C₁₀ alkenyl, optionally substituted C₂-C₁₀ alkynyl, optionally substituted C₃-C₁₀ cycloalkyl, optionally substituted C₆-C₁₀ aryl or a 5-10-membered, optionally substituted heteroaryl group, wherein the optional substituents of the alkyl group are selected from halogen, hydroxyl, amino and C₁-C₄ alkoxy, and the optional substituents of the other groups are selected from halogen, hydroxyl, amino, C₁-C₄ alkyl and C₁-C₄ alkoxy.

As preferred examples, glyoxylic acid, pyruvic acid and 2-ketobutyric acid can be mentioned.

The carboxylic acid of Formula (I) may be comprised in the composition in its free acid form. The carbonyl group adjacent to the acid group of the acid may also be present in the hydrate form. Apart from the free acid form and the hydrate thereof, salts of the acid or the hydrate may also be used.

The hydrate of the acid of Formula (I) may be formed when providing the composition in an aqueous medium. For instance, glyoxylic acid (H-CO-COOH) in aqueous solution is almost quantitatively present as the hydrate (H-C(OH)₂-COOH). Besides, the hydrate may also condense to dimers.

Salts of the carboxylic acid of Formula (I) may also be used. As examples, alkali metal salts such as the sodium or potassium salts, alkaline earth metal salts such as magnesium salt or the calcium salt and tertiary and quaternary ammonium salts may be mentioned. In the present invention, glyoxylic acid, its salts, and its hydrated form are the more preferred carboxylic acids of Formula (I).

The concentration of the at least one carboxylic acid of the Formula (I) and/or a hydrate thereof and/or salts thereof is in the range of 0.1 to 40%, preferably 0.5 to 30%, more preferably 1 to 25% and more preferably 2.5 to 20%, and even more preferably 2.5 to 14% by weight, calculated to the total of the composition.

The composition comprises one or more amino acids. All amino acids are suitable for the purposes of the present invention. The amino acids are glycine, alanine, leucine, arginine, aspartate, asparagine, cysteine, glutamine, glutamate, histidine, isoleucine, lysine, methionine, phenylalanine, proline, serine, threonine, tryptophan, tyrosine, and valine. Preferred are glycine, alanine, leucine, isoleucine, proline, serine, threonine, tryptophan, tyrosine, and arginine. More preferred are glycine, alanine, leucine, isoleucine, proline, serine, tyrosine, and arginine. Most preferred are glycine, alanine, and leucine. The total concentration of one or more amino acids is in the range of 0.001 to 15%, preferably 0.01 to 12.5%, more preferably 0.02 to 10%, and most preferably 0.1 to 5% by weight, calculated to the total of the composition.

The pH of the composition is 4.0 or less. It is in the range of 1 to 4 and preferably 1.5 to 3.5, more preferably 1.8 to 3.0 and most preferably 1.8 to 2.5. The pH is measured at ambient temperature i.e. at approximately 20°C using a suitable commercially available suitable pH meter.

As mentioned above, the conventional techniques are based on the re-organization of the disulfide bridges and involve a cleavage of the disulfide bonds by using a sulfur-based reducing agent, followed by the shaping of the hair and the formation of new disulfide bonds by the action of an oxidizing agent.

In contrast, the present invention does not utilize cleavage of the disulfide bonds. The straightening composition of the present invention, therefore, does not require the presence of sulfur-based reducing agents, and is preferably is free of sulfur-based reducing agents. Up to 2% by weight, calculated to the total of the composition sulfur based reducing agents, however, does not disturb the straightening performance of the compositions. Consequently, there is also no need to re-oxidize the disulfide bonds and, therefore, there is no need of application of an additional composition comprising oxidizing agent.

In an embodiment of the present invention, the composition comprises one or more thickeners. The thickener polymers are preferably selected from non-ionic, anionic, cationic, and amphoteric polymers, having a viscosity of at least 500 mPa·s measured at a polymer concentration of 1% by weight in water, and at 20°C with a Brookfield viscometer at 10 rpm for 1 minute with an appropriate spindle.

The total concentration of the one or more thickening agents is typically within the range of 0.01 to 15 wt.%, preferably 0.05 to 10 wt.%, more preferably 0.1 to 5 wt.%, and more preferably 0.5 to 2 wt.% based on the weight of the straightening composition. Principally, any cosmetically acceptable thickening polymer of above referred ionicity may be comprised in the compositions. The composition may include a single kind or multiple kinds of polymers, with the same ionicity or may comprise mixture of several polymer types.

Suitable examples for the non-ionic polymers include neutral polysaccharides and derivatives such as ethers or esters thereof. In this respect, neutral gums such as guar gum, hydroxypropyl guar, cellulose ethers such as hydroxyethylcellulose (HEC), methyl hydroxyethylcellulose (MHEC), ethyl hydroxyethylcellulose (EHEC), methyl ethyl hydroxyethylcellulose (MEHEC), hydroxypropylcellulose (HPC), hydroxypropylmethylcellulose (HPMC), hydrophobically modified derivatives thereof such as HM-EHEC, starch, and dextrins may be mentioned.

Examples for the anionic polymer include anionic polysaccharides and derivatives thereof, such as alginate, pectin, hyaluronate, anionic gums such as xanthan gum, dehydroxanthan gum, hydroxypropyl xanthan gum, gum arabic, gum karaya or gum tragacanth, or anionic cellulose derivatives such as carboxymethyl cellulose (CMC). Further examples include synthetic anionic polymers such as polyacrylic acid or copolymers containing acrylic acid in combination with neutral vinylic and/or acrylic monomers, and salts thereof such as sodium polyacrylate.

Xanthan gum, hydroxypropyl xanthan gum and dehydroxanthan gum are particularly preferable in view of pH stability. In an embodiment of the present invention, therefore, the composition comprises Xanthan gum and/or hydroxypropyl xanthan gum and/or dehydroxanthan gum.

Suitable cationic polymers are known with the CTFA adopted name Polyquaternium and any of these known, and allowed, in cosmetic may be suitable for the compositions of the present invention. Suitable non limiting examples are Polyquaternium-6, Polyquaternium-7, Polyquaternium-10, Polyquaternium-37 and Polyquaternium-67.

As suitable examples for the amphoteric polymer, carboxyl-modified or sulfonate-modified cationic polysaccharides such as carboxymethylchitosan may be mentioned.

Further examples include copolymers of cationic vinylic or (meth)acrylic monomers with (meth)acrylic acid, such as dimethyldiallylammonium chloride/acrylic acid copolymer (polyquaternium-22).

As an amphoteric polymer, polyquaternium-22 is particularly preferable.

The straightening composition may comprise a surfactant. As the surfactant, any of a cationic surfactant, a nonionic surfactant, an amphoteric surfactant, and an anionic surfactant can be used. It is also possible to use two or more types of surfactants in combination.

The cationic surfactant is preferably a mono-long chain alkyl quaternary ammonium salt, having a C₈-C₂₄ alkyl residue and three C₁-C₄ alkyl residues.

Preferably at least one mono alkyl quaternary ammonium surfactant is selected from the compounds with the general formula 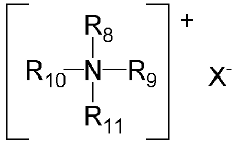
wherein R₈ is a saturated or unsaturated, branched, or straight alkyl chain with 8-22 C atoms or

   R₁₂-CO-NH-(CH₂)ₙ-
wherein R₁₂ is a saturated or unsaturated, branched, or straight alkyl chain with 7-21 C atoms and n is an integer of 1 - 4, or

   R₁₂-CO-O-(CH₂)ₙ-
wherein R₁₂ is a saturated or unsaturated, branched, or straight alkyl chain with 7-21 C atoms and n is an integer of 1 - 4, and
R₉, R₁₀ and R₁₁ are independent from each other alkyl group with 1 to 4 carbon atoms, hydroxyl alky chain with 1 to 4 carbon atoms, or ethoxy or propoxy group containing any number of ethoxy or propoxy groups, n is varying in the range of 1 to 4, and X is chloride, bromide, methosulfate or ethosulfate.

Suitable cationic surfactants are, for example, long-chain quaternary ammonium compounds which can be used alone or in admixture with one another, such as cetyl trimethyl ammonium chloride, myristyl trimethyl ammonium chloride, behentrimonium chloride, trimethyl cetyl ammonium bromide, stearyl trimethyl ammonium chloride, stearyl trimonium chloride, and stearamidopropyltrimonium chloride.

Examples of the nonionic surfactant include polyoxy-C₁₋₄-alkylene C₈₋₂₄-alkyl ether, polyoxy-C₁₋₄-alkylene C₈₋₂₄-alkylene alkenyl ether, higher (C₁₂-C₂₄) fatty acid sucrose ester, polyglycerin C₈₋₂₄-fatty acid ester, higher (C12-C24) fatty acid mono- or diethanolamide, polyoxyethylene hardened castor oil, polyoxyethylene sorbitan C₈₋₂₄-fatty acid ester, polyoxyethylene sorbit C₈₋₂₄-fatty acid ester, C₈₋₂₄-alkyl saccharide surfactant, C₈₋₂₄-alkylamine oxide, and C₈₋₂₄-alkylamidoamine oxide.

Examples of the amphoteric surfactant include an imidazoline-based surfactant, a carbobetaine-based surfactant, an amidobetaine-based surfactant, a sulfobetaine-based surfactant, a hydroxysulfobetaine-based surfactant, and an amidosulfobetaine-based surfactant.

Examples of the anionic surfactant include alkylbenzenesulfonate, alkyl or alkenyl ether sulfate, alkyl or alkenyl sulfate, olefin sulfonate, alkanesulfonate, saturated or unsaturated fatty acid salts, alkyl, or alkenyl ether carboxylate, α-sulfo fatty acid salts, N-acylamino acid type surfactants, phosphoric acid mono- or diester type surfactants, and sulfosuccinate. Examples of the alkyl ether sulfate include polyoxyethylene alkyl ether sulfate. Examples of the counterion for the anionic residues of these surfactants include an alkali metal ion such as a sodium ion or potassium ion; an alkaline earth metal ion such as a calcium ion or magnesium ion; an ammonium ion; and an alkanolamine having 1 to 3 alkanol groups each having 2 or 3 carbon atoms (for example, monoethanolamine, diethanolamine, triethanolamine, or triisopropanolamine).

The surfactant can be used singly or in combination of two or more kinds. When adding a surfactant to the straightening composition, the content thereof is usually 0.05 to 10% wt.%, more preferably 0.1 to 5 wt.%, based on the total weight of the straightening composition.

In an embodiment of the present invention, the compositions may comprise one or more oil selected from silicone oils, synthetic oils, and natural triglycerides.

Suitable silicone oils are those especially known with the CTFA adopted name dimethicone with carious viscosities such as the ones commercially available under the trade name Xiameter, amodimethicones, cyclic silicones such as cyclomethicone, polyalkylene group modified silicones, and aminated silicones known by the CTFA adopted name Polysilicone from the present applicant. The compositions may comprise single, as well as combinations of, silicones.

Synthetic oils other than the silicones are fatty acid esters such as isopropyl isostearate, hexyl laurate, isohexyl laurate, isohexyl palmitate, isopropyl palmitate, isopropyl myristate, decyl oleate, isodecyl oleate, hexadecyl stearate, decyl stearate, dihexadecyl adipate, lauryl lactate, myristyl lactate, cetyl lactate, oleyl stearate, oleyl oleate, oleyl myristate, lauryl acetate, cetyl propionate and dioleyl adipate.

Further suitable oil components are natural oils, such as paraffin oil and natural triglycerides.

Suitable natural triglycerides are argan oil, shea butter oil, karite oil, olive oil, almond oil, avocado oil, ricinus oil, coconut oil, palm oil, sesame oil, peanut oil, sunflower oil, peach kernel oil, wheat germ oil, macadamia nut oil, macadamia oil, night primrose oil, jojoba oil, castor oil, soya oil, lanolin, passiflora oil, black cumin oil, borage oils, grapeseed oil, hempseed oil, kukui nut oil, and rosehip oil.

The one or more oils are comprised at a total concentration in the range of 0.1 to 20 wt.%, preferably 0.2 to 10 wt.%, more preferably 0.5 to 5 wt.%, calculated to the total of the composition.

The compositions may comprise additionally compounds customarily found in such formulations such as preservatives, fragrances, solvents such as monohydric alcohols i.e., ethanol, isopropanol.

The following examples are to illustrate the invention but not to limit it.

### Example 1

The following compositions were prepared, and tensile measurements were carried out to show the effect of the invention.

| | | | % by weight | | | | | | | | | |
|---|---|---|---|---|---|---|---|---|---|---|---|---|
| | A | B | C | D | E | F | G | H | I | J | K | L |
| Glyoxylic acid | 10 | 10 | 10 | 10 | 10 | 10 | 10 | 10 | 10 | 10 | 10 | 10 |
| Glycine | - | 0,5 | 1 | 3 | 5 | 10 | - | - | - | - | - | - |
| Leucine | - | - | - | - | - | - | 0,5 | 5 | 10 | - | - | - |
| Alanine | - | - | - | - | - | - | - | - | - | 0,5 | 5 | 10 |
| Sodium hydroxide | | q.s. to pH 2.0 | | | | | | | | | | |
| Water | | q.s. to 100 | | | | | | | | | | |

The compositions were prepared by dissolving the ingredients in water and the pH of the composition was adjusted using sodium hydroxide (32%) solution.

The hair streaks, weighing approximately 2 g and having a length of 30 cm (Indian Frizz Hair, bought from International Hair Importers & Products Inc., NY), were straightened with the above compositions in the following way.

The hair was shampooed with a commercially available shampoo, under the brand Goldwell, towel dried and then 2 g of the above composition was applied onto hair (hair to composition ratio was 1:1 by weight). The composition was distributed homogeneously by massaging and left on the hair for 15 min. Afterwards, the streaks were dried with a hair drier and treated with a flat iron having a surface temperature of 230°C by passing through the hair streak 6 times, for a total of 30 sec. This was followed by washing the hair with shampoo and drying with a hair drier. It was observed that all hair streaks were straightened satisfactorily. For each composition one hair streak was used. Tensile measurements were carried out using single fiber tensile tester, Mtt675 & Mtt680 with incorporated high frequency laser FDAS675 for cross-section measurement (DiaStron limited, UK) at 20°C, 100%RH. For each hair streak, at least 6 measurements (*I did only 1x measurement for each streak) were carried out with the use of 30 single fibers per streak.

In addition to the above treated streaks, a streak treated with flat iron only, as described above, was also subjected to tensile measurement.

The following results were obtained.

| | |
|---|---|
| Composition | 2% Index |
| Untreated | 43,36 |
| Iron Treated | 40,14 |
| A | 29,88 |
| B | 36,35 |
| C | 34,06 |
| D | 32,89 |
| E | 33,00 |
| F | 35,58 |
| G | 34,59 |
| H | 35,95 |
| I | 35,61 |
| J | 34,13 |
| K | 34,53 |
| L | 36,42 |

From the above results, it is beyond any doubt that treating hair with glyoxylic acid solution not comprising any amino acid results in a loss of 2% index (Iron treated vs A). The 2% index is considerably less reduced when the composition comprises an amino acid at various concentrations (Compositions B to L in the above Table).

Similar results were observed with the following Examples

### Example 2

| | |
|---|---|
| Glyoxylic acid | 10.0 |
| Glycine | 1.0 |
| Cetrimonium chloride | 2.0 |
| Sodium hydroxide 32% | q.s. to pH 2.2 |
| Water | q.s. to 100 |

### Example 3

| | |
|---|---|
| Glyoxylic acid | 10.0 |
| Glycine | 1.0 |
| Dehydroxanthan gum | 2.0 |
| Sodium hydroxide 32% | q.s. to pH 2.2 |
| Water | q.s. to 100 |

### Example 4

| | |
|---|---|
| Glyoxylic acid | 10.0 |
| Glycine | 1.0 |
| Dehydroxanthan gum | 2.0 |
| Dimethicone | 2.0 |
| Sodium hydroxide 32% | q.s. to pH 2.2 |
| Water | q.s. to 100 |

### Example 5

| | |
|---|---|
| Glyoxylic acid | 10.0 |
| Glycine | 1.0 |
| Cetrimonium chloride | 2.0 |
| Cetearyl alcohol | 5.0 |
| Dimethicone | 2.0 |
| Sodium hydroxide 32% | q.s. to pH 2.2 |
| Water | q.s. to 100 |

## Claims

1. An aqueous composition for semipermanent straightening keratin fibers, especially human hair, **characterized in that** it comprises at least one carboxylic acid of the formula (I) and/or a hydrate thereof and/or a salt thereof:
R-CO-COOH Formula (I)
wherein R is selected from hydrogen, COOH, CN, optionally substituted C₁-C₁₀ alkyl, optionally substituted C₂-C₁₀ alkenyl, optionally substituted C₂-C₁₀ alkynyl, optionally substituted C₃-C₁₀ cycloalkyl, optionally substituted C₆-C₁₀ aryl or a 5-10-membered, optionally substituted heteroaryl group, wherein the optional substituents of the alkyl group are selected from halogen, hydroxyl, amino and C₁-C₄ alkoxy, and the optional substituents of the other groups are selected from halogen, hydroxyl, amino, C₁-C₄ alkyl and C₁-C₄alkoxy; and
one or more amino acid, and it has a pH 4 or less.

2. The composition according to claim 1 **characterized in that** it is a semi-permanent hair straightening composition.

3. The composition according to claims 1 and/or 2 **characterized in that** it comprises glyoxylic acid and/or hydrates thereof and/or salts thereof and or their mixtures as the carboxylic acid according to the formula I.

4. The composition according to any of the preceding claims **characterized in that** it comprises at least one carboxylic acid of the Formula (I) and/or a hydrate thereof and/or salts thereof at a concentration in the range of 0.1 to 40%, preferably 0.5 to 30%, more preferably 1 to 25% and most preferably 2.5 to 20% and in particular 2.5 to 14%, by weight, calculated to the total of the composition.

5. The composition according to any of the preceding claims **characterized in that** one or more amino acids is selected from glycine, alanine, leucine, arginine, aspartate, asparagine, cysteine, glutamine, glutamate, histidine, isoleucine, lysine, methionine, phenylalanine, proline, serine, threonine, tryptophan, tyrosine and valine, preferably it is selected from glycine, alanine, leucine, isoleucine, proline, serine, threonine, tryptophan, tyrosine and arginine, more preferably it is selected from glycine, alanine, leucine, isoleucine, proline, serine, tyrosine and arginine and most preferably it is selected from glycine, alanine and leucine.

6. The composition according to any of the preceding claims **characterized in that** it comprises one or more amino acids at a total concentration in the range of 0.001 to 15%, preferably 0.01 to 12.5%, more preferably 0.02 to 10%, and most preferably 0.1 to 10% by weight calculated to the total of the composition.

7. The composition according to any of the preceding claims **characterized in that** it has a pH in the range of 1 to 4, preferably 1.5 to 3.5, more preferably 1.8 to 3.0 and most preferably 1.8 to 2.5.

8. The composition according to any of the preceding claims **characterized in that** it comprises one or more thickener, preferably selected from non-ionic, anionic, cationic and amphoteric polymers having a viscosity of at least 500 m Pa·s measured at a polymer concentration of 1% by weight in water and at 20°C with a Brookfield viscometer at 10 rpm for 1 minute with an appropriate spindle.

9. The composition according to any of the preceding claims **characterized in that** it comprises one or more surfactants, preferably selected from cationic, anionic, non-ionic and amphoteric surfactants.

10. The composition according to any of the preceding claims **characterized in that** it comprises one or more fatty alcohol.

11. The composition according to any of the preceding claims **characterized in that** it comprises one or more oil selected from silicone oils, synthetic oils, and natural triglycerides.

12. The composition according to any of the preceding claims **characterized in that** it is a two-part composition comprising Part A and B and mixed immediately before application onto hair into a homogeneous composition, wherein the Part A comprises at least one carboxylic acid of the formula (I) and/or a hydrate thereof and/or a salt thereof and Part B comprises ectoin and/or hydroxyectoin.

13. The composition according to any of the preceding claims **characterized in that** it does not comprise reducing agent and/or oxidizing agent.

14. Method of straightening keratin fibers especially human hair **characterized in that** it comprises the steps of
- application of the composition according to any of the claims 1 to 11,
- leaving on the hair for a period of 1 to 120 min,
- optionally rinsing off the hair,
- drying the hair,
- treating the hair with an iron having a surface temperature of 180 ± 50°C; and
- optionally rinsing off the hair and/or shampooing the hair and drying.

15. Kit for semipermanent straightening keratin fibers, especially human hair it comprises the composition according to claims 1 to 11 and 13 or the compositions part A and B according to claims 11 and 12 and a straightening iron.
